(19) Europäisches Patentamt
European Patent Office
Office européen des brevets

(11) Veröffentlichungsnummer: **0 305 829**
**A2**

# (12) EUROPÄISCHE PATENTANMELDUNG

(21) Anmeldenummer: **88113543.8**

(22) Anmeldetag: **20.08.88**

(51) Int. Cl.[4]: **C07C 29/32 , C07C 31/08 , C07C 31/10 , C07C 31/12**

(30) Priorität: **29.08.87 DE 3728981**

(43) Veröffentlichungstag der Anmeldung: **08.03.89 Patentblatt 89/10**

(84) Benannte Vertragsstaaten: **AT BE DE FR GB IT NL SE**

(71) Anmelder: **HOECHST AKTIENGESELLSCHAFT**
**Postfach 80 03 20**
**D-6230 Frankfurt am Main 80(DE)**

(72) Erfinder: **Strohmeier, Walter, Prof. Dr. rer. nat.**
**Mittl. Dallenbergweg 21**
**D-8700 Würzburg(DE)**

## (54) Verfahren zur Herstellung von Ethanol im Gemisch mit Propanol und Butanol.

(57) Die Erfindung betrifft ein Verfahren zur Herstellung von Ethanol, Propanol und Butanol aus Methanol, Kohlenmonoxid und Wasserstoff. Die Umsetzung erfolgt in Gegenwart eines Katalysators, der Kobalt und Ruthenium, Jod oder Jodid und ein organisches Monophosphan oder Phosphit oder ein zweizähniges organisches Phosphan oder Phosphit enthält. Erfindungsgemäß wird dem Reaktionsgemisch ein cyclischer Mono- oder Polyether zugesetzt.

EP 0 305 829 A2

## Verfahren zur Herstellung von Ethanol im Gemisch mit Propanol und Butanol

Die Erfindung betrifft ein Verfahren zur Herstellung von Ethanol und daneben n-Propanol und n-Butanol durch Umsetzung von Methanol mit Kohlenmonoxid und Wasserstoff in Gegenwart von Kobalt und Ruthenium enthaltenden Katalysatoren. Diese Reaktion wird als Homologisierung bezeichnet. Sie ermöglicht es, ausgehend vom einfachsten Alkohol, durch Einführung einer oder mehrerer $CH_2$-Gruppen höhere homologe Alkohole herzustellen.

Die Homologisierung findet im verstärkten Maße Interesse, da sie einen Weg zur Gewinnung höherer Alkohole eröffnet, der unabhängig von der Verwendung von Erdöl ist. Als Einsatzstoffe werden Synthesegas und daraus hergestelltes Methanol benötigt; Synthesegas ist z.B. aus Kohle oder Erdgas nach verschiedenen, technisch bewährten Prozessen, zugänglich.

Es ist seit langem bekannt (vgl. z.B. DE-PS 867 849), Methanol mit Wasserstoff und Kohlenmonoxid in Gegenwart eines wasserlöslichen Kobaltkatalysators bei hohen Temperaturen und Drücken in Ethanol umzuwandeln. Diese Reaktion verläuft nach der Gleichung:

$$CH_3OH + CO + 2H_2 \rightarrow C_2H_5OH + H_2O$$

daneben können in untergeordnetem Maße auch höhere Alkohole entsprechend

$$CH_3OH + n(CO + 2H_2) \rightarrow CH_3(CH_2)_nOH + n\ H_2O$$

gebildet werden.

Während ursprünglich für die Reaktion ausschließlich Kobalt als Katalysator verwendet wurde, gewannen im Laufe der Zeit Mehrkomponenten-Katalysatoren zunehmend Bedeutung.

In der US-PS 32 85 948 ist die Herstellung von Ethanol aus Methanol unter Verwendung von Kobalt als Katalysator, Jod oder einer Jodverbindung als erstem und einem Rutheniumhalogenid oder einem Osmiumchlorid als weiterem Promotor beschrieben. Die beanspruchten Maßnahmen sollen zu einer Erhöhung der Selektivität der Reaktion zu Ethanol führen.

Das gleiche Ziel wird nach dem Verfahren der DE-OS 26 25 627 mit einem Katalysatorsystem angestrebt, das aus Kobalt, einem Halogenid als Promotor und einem tertiären Phosphan besteht; die Umsetzung erfolgt in einem Kohlenwasserstoff als Lösungsmittel.

Nach der Lehre der US-PS 41 33 966 gewinnt man Ethanol aus Methanol und Synthesegas unter Verwendung eines aus Kobaltacetylacetonat, einer organischen Verbindung eines Elementes der Gruppe VA des Periodensystems der Elemente, einer Rutheniumverbindung und einer Jodverbindung bestehenden Katalysators.

Die vorstehend beschriebenen Maßnahmen reichen nicht aus, die Selektivität der Reaktion soweit zu steigern, daß sie für eine technische Anwendung geeignet ist. Hierbei ist zu berücksichtigen, daß Nebenprodukte nicht nur in großer Menge, sondern in Form zahlreicher, unterschiedlicher Einzelverbindungen anfallen. So werden neben den erwünschten Alkoholen auch Kohlenwasserstoffe wie Methan, Ethan, Propan, verschiedene Ether, ferner Ester, z.B. Methylacetat, Ethylacetat, Propylacetat und Acetale wie Acetaldehyd-dimethylacetal, Acetaldehyd-methylethylacetal und Acetaldehyd-diethylacetal gebildet. Die industrielle Nutzung des Prozesses erfordert daher einen hohen, wirtschaftlich nicht immer vertretbaren Aufwand, um aus der Vielfalt der im Reaktionsgemisch enthaltenen Verbindungen auf physikalischem oder chemischem Wege diejenigen Substanzen zu isolieren, die als Zwischen- und Endprodukte Verwendung finden können.

Zwar läßt sich die Selektivität der Umsetzung durch Zusatz eines Lösungsmittels zu den Reaktionspartnern verbessern, diese Maßnahme hat jedoch eine erhebliche Abnahme des Umsatzes, bezogen auf Reaktorvolumen und Zeit, zur Folge.

Eine Reduzierung der Nebenproduktbildung ohne erhebliche Beeinträchtigung des Umsatzes erreicht man durch Einsatz bestimmter Phosphane als Bestandteil des Katalsatorsytems. So wird in der DE 30 42 434 Al ein Verfahren zur Herstellung von Ethanol und n-Propanol beschrieben, bei dem Methanol mit Kohlenmonoxid und Wasserstoff bei 150 bis 250 °C und erhöhtem Druck umgesetzt wird. Die Reaktion erfolgt in Gegenwart von Katalysatoren, die Kobalt und Ruthenium als Verbindung, Jod oder ein Jodid und ein zweizähniges organisches Phosphan oder Phosphit enthalten.

Obgleich diese Variante der Homologisierung von Methanol zu einer deutlichen Verbesserung des Methanolumsatzes und der Selektivität der Reaktion führt, besteht weiterhin die Aufgabe, den Prozeß hinsichtlich beider Merkmale zu verbessern.

Erfindungsgemäß wird dieses Ziel erreicht mit Hilfe eines Verfahrens zur Herstellung von Ethanol im Gemisch mit Propanol und Butanol durch Umsetzung von Methanol mit Kohlenmonoxid und Wasserstoff bei Temperaturen von 180° bis 250° C und Drücken von 20 bis 60 MPa in Gegenwart von 0,07 bis 0,4 Mol Wasser je Mol Methanol und von Katalysatoren, die Kobalt und Ruthenium in elementarer Form oder in Form von Verbindungen, Jod oder Jodid und ein organisches Monophosphan oder Phosphit oder ein zweizähniges organisches Phosphan oder Phosphit enthalten. Es ist dadurch gekennzeichnet, daß dem Reaktionsgemisch je Mol Methanol 0,2 bis 4 Mol eines cyclischen Mono- oder Polyethers zugesetzt werden.

Charakteristisch für das erfindungsgemäße Verfahren ist der Zusatz eines cyclischen Mono- oder Polyethers zum Reaktionsgemisch. Überraschenderweise bewirken diese Ether, daß Methanolumsatz und Selektivität der Reaktion im Vergleich zu den Verfahren des Standes der Technik, weiter gesteigert werden können. Insbesondere die Entstehung von Ethern wird deutlich zurückgedrängt. Die cyclischen Mono- oder Polyether werden im allgemeinen als einheitliche Verbindung eingesetzt, jedoch ist es selbstverständlich auch möglich, Gemische aus zwei oder mehr Verbindungen zu verwenden. Besonders bewährt als cyclische Mono- oder Polyether haben sich Tetrahydrofuran und Dioxan.

Ein wesentlicher Aspekt der neuen Arbeitsweise ist, daß die Etherbildung nur dann weitgehend unterbleibt, wenn der cyclische Mono- oder Polyether zusammen mit Katalysatoren eingesetzt wird, die organische Monophosphane oder Phosphite oder zweizähnige Phosphane oder Phosphite enthalten. Darüber hinaus ist es möglich, durch Variation der Reaktionsbedingungen die Anteile Ethanol, Propanol und Butanol im Reaktionsprodukt zu beeinflussen.

Je Mol Methanol setzt man 0,2 bis 4 Mol des im Reaktionsgemisch löslichen cyclischen Ethers ein. Bewährt hat es sich, je Mol Methanol 0,3 bis 3 und insbesondere 0,5 bis 1,5 Mol cyclischen Ethers zu verwenden.

Die für das erfindungsgemäße Verfahren eingesetzten Katalysatoren enthalten Kobalt und Ruthenium, ferner Jod oder Jodid und weiterhin ein organisches Monophosphan oder Phosphit oder ein zweizähniges organisches Phosphan oder Phosphit.

Kobalt und Ruthenium werden entweder elementar oder als Verbindungen eingesetzt. Sie wandeln sich unter den Reaktionsbedingungen in Gegenwart von Kohlenmonoxid und Wasserstoff in Carbonyl- oder Hydrocarbonylverbindungen um. Bei Verwendung in elementarer Form geht man zweckmäßig von den sehr feinteiligen Metallen aus, um sicherzustellen, daß sie schnell und vollständig in die katalytisch aktiven Carbonylkomplexe überführt werden.

Kobaltverbindungen, die dem Reaktionsgemisch zugesetzt werden können, sind Salze wie Kobalt-2-ethylhexanoat, Kobaltacetylacetonat, Kobalthalogenide, Kobaltnitrat, ferner Kobaltoxid oder Kobalthydroxid. Besonders bewährt hat sich Kobaltcarbonat. Ruthenium wird ebenfalls in Form der Halogenide, des 2-Ethylhexanoats, des Ethylacetonats, vorzugsweise des Chlorids, $RuCl_3 \cdot XH_2O$ verwendet. Auch binukleare Ruthenium-Komplexverbindungen wie $[NH_4]_4(Ru_2OCl_{10})$ sind geeignet. Selbstverstandlich können Kobalt und Ruthenium auch als Carbonylverbindungen oder deren Derivate, wie $Co_2(CO)_6L_2$ (mit L= organisches Phosphan oder Phosphit) oder $RuX_2(CO)_2L_2$ (mit X= Halogen und L= organisches Phosphan oder Phosphit) eingesetzt werden.

Weitere Bestandteile der Katalysatoren sind organische Monophosphane oder zweizähnige organische Phosphane oder Phosphite der allgemeinen Formel

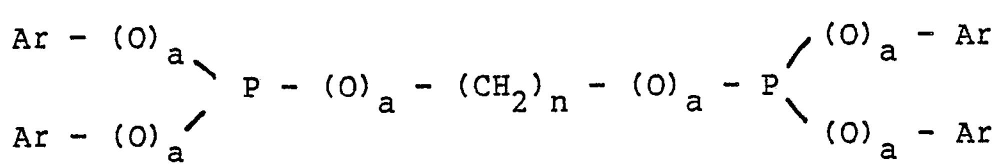

Hierbei ist Ar ein Arylrest, insbesondere der Phenylrest, a bedeutet 0 oder 1 und n eine ganze Zahl von 1 bis 6. Beispiele für organische Monophosphane sind die Verbindungen Tributylphosphan, Triphenylphosphan, Tri-p-tolylphosphan und Trimethoxiphosphan. Beispiele für Verbindungen nach der allgemeinen Formel, die im Rahmen der erfindungsgemäßen Arbeitsweise eingesetzt werden können, sind 1,2-Bis-(diphenylphosphino)methan, 1,3-Bis(diphenylphosphino)ethan, 1,3-Bis(diphenylphosphino)propan, 1,4-Bis-(diphenylphosphino)butan. Besonders bewährt hat sich 1,3-Bis(diphenylphosphino)propan.

Schließlich enthält das Katalysatorsystem noch Jod in molekularer oder in ionischer Form. Als Salze der Jodwasserstoffsäure können Alkalimetalljodide und besonders vorteilhaft Kobaltjodid verwendet werden.

Das im erfindungsgemäßen Verfahren verwendete Katalysatorsystem wird dem Reaktionsgemisch

üblicherweise in Form seiner einzelnen Bestandteile zugeführt. Eine Präformierung der Metallkomplexverbindungen, die Komponenten des Katalysatorsystems sind, ist zwar möglich und in Einzelfällen angeraten aber nicht zwingend erforderlich. Um eine Desaktivierung des Katalysators zu vermeiden, ist sowohl bei seiner Herstellung als auch bei Lagerung und Verwendung auf den Ausschluß von Luft zu achten.

Das Katalysatorsystem ist wiederholt, ohne Desaktivierung einsetzbar, wenn die Reaktionsprodukte und der cyclische Mono- oder Polyether unter Luftausschluß abgetrennt werden. Bei wiederholtem Einsatz des Katalysatorsystems nimmt seine Aktivität sogar zu, wenn den Ansätzen jeweils 0,2 bis 5 Gew.-%, vorzugsweise 0,5 bis 1 Gew.-% des ursprünglich als Metall oder Verbindung eingesetzten Kobalts zugefügt werden.

Methanol wird üblicherweise in Form des in technischen Anlagen hergestellten Produktes mit einem Wassergehalt von 4 bis 6 % verwendet. Eine zusätzliche Reinigung ist nicht erforderlich.

Das ursprünglich eingesetzte Reaktionsgemisch enthält je Mol Methanol 0,07 bis 0,4 Mol Wasser. Besonders bewährt hat es sich, je Mol Methanol 0,1 bis 0,3 mol Wasser anzuwenden. Der Wasserzusatz bewirkt eine Erhöhung des Umsatzes. Höhere Wassermengen beeinflussen den Umsatz nur unwesentlich, geringere Mengen haben keine oder nur unerhebliche Umsatzsteigerungen zur Folge. Zweckmäßig wird das Wasser zusammen mit dem Methanol dem Reaktor zugeführt.

Je g-Atom Kobalt setzt man 20 bis 10.000, vorzugsweise 50 bis 5.000 und insbesondere 200 bis 2.000 Mol Methanol ein.

Kobalt und Phosphan bzw. Phosphit werden in einem molaren Verhältnis von 1 : 0,1 bis 1 : 20, vorzugsweise 1 : 0,1 bis 1 : 5 und insbesondere 1 : 0,33 bis 1 : 2 eingesetzt.

Durch Erhöhung des Co/Phosphan-Verhältnisses begünstigt man die Bildung von Propanol und Butanol zu Lasten des Ethanolanteils im Reaktionsprodukt. So ergeben $CoJ_2$/Phosphan im Molverhältnis 1 : 1 nur etwa 16 Gew.-% Propanol und Butanol, bezogen auf die Alkohole im Reaktionsprodukt, während bei einem Molverhältnis von 3 : 1 66 Gew.-% Propanol und Butanol erhalten werden.

Das Atomverhältnis von Kobalt zu Ruthenium beträgt 1 : 0,0005 bis 1 : 1, vorzugssweise 1 : 0,05 bis 1 : 0,5 und insbesondere 1 : 0,1 bis 1 : 0,3.

Je g-Atom Kobalt sind in den Katalysatoren 0,002 bis 3, vorzugsweise 0,1 bis 2,5 und insbesondere 0,5 bis 2 Mol Jod enthalten.

Das molare Verhältnis von Kohlenmonoxid und Wasserstoff im Synthesegas beträgt üblicherweise etwa 1 : 2, doch kann Wasserstoff auch im Überschuß über dieses Verhältnis vorhanden sein. Mischungen, die CO und $H_2$ im Verhältnis 1 : 3 bis 1 : 4 enthalten, können ohne Nachteile eingesetzt werden.

Das Kohlenmonoxid-/Wasserstoff-Gemisch soll keine, die Aktivität des Katalysatorsystems beeinflussenden Verunreinigungen, wie Schwefelverbindungen und Cyanwasserstoff, enthalten. Kohlendioxid und/oder Stickstoff bis zu 5 Vol.-%, bezogen auf das Gesamtgemisch, schaden nicht.

Der neue Prozeß kann sowohl diskontinuierlich als auch kontinuierlich durchgeführt werden. Im allgemeinen erfolgt die Umsetzung von Methanol, Kohlenmonoxid und Wasserstoff bei Temperaturen von 180 bis 250 °C, vorzugsweise 180 bis 230 °C und insbesondere 200 bis 220 °C. Der Druck wird auf Werte zwischen 20 bis 60 MPa, vorzugsweise 45 bis 60 MPa und insbesondere 40 bis 50 MPa eingestellt. Das molare Verhältnis von Methanol zu Synthesegas, also $CH_3OH$ zu ($CO+H_2$), wobei das $CO/H_2$-Gemisch wie oben angegeben zusammengesetzt ist, kann sowohl bei kontinuierlicher als auch bei absatzweiser Reaktionsführung 1 : 0,1 bis 1 : 20, vorzugsweise 1 : 0,5 bis 1 : 5 betragen.

Die Verweilzeit der Reaktanten im Reaktor beträgt bis zu etwa 8 Stunden, vorzugsweise ist sie 4 bis 8 und insbesondere 3 bis 6 Stunden.

In den nachstehenden Ausführungsbeispielen wird die Erfindung erläutert ohne sie in ihrem Schutzumfang zu beschränken.

Die Versuche werden in Autoklaven durchgeführt, die mit Gaseinlaß und -auslaß, Thermofühler, Magnetrührer und Heizmantel ausgestattet sind.

Katalysatorsystem und Methanol-Wasser-Gemisch werden bei Raumtemperatur unter Luft in die Autoklaven gefüllt. Die Luft wird sofort durch mehrfaches Aufdrücken von Synthesegas bis 2,0 MPa und vorsichtiges Entspannen verdrängt. Danach wird Synthesegas bis zu einem Druck von 5,0 MPa aufgepreßt, auf die gewünschte Reaktionstemperatur aufgeheizt und der gewünschte Reaktionsdruck eingestellt.

In den Versuchen werden sofern nichts anderes angegeben ist

0,2 mmol $CoI_2$
0,04 mmol $RuI_2(CO)_2(PØ_3)_2$
0,2 mmol NaI
20 mmol $H_2O$
200 mmol Methanol

eingesetzt (Standardansatz). Die weiteren Reaktionsteilnehmer und die Reaktionsbedingungen sind den tabellarischen Zusammenstellungen in den Beispielen zu entnehmen. Das Synthesegas enthält je Volumen Kohlenmonoxid 2 Volumina Wasserstoff.

Nach Ablauf der vorgegebenen Reaktionszeit wird der Autoklav auf 0 °C gekühlt und langsam auf Normaldruck entspannt. Zur gaschromatografischen Analyse wird jeweils eine Probe des abgekühlten homogenen Reaktionsgemisches entnommen.

Der in den Versuchsauswertungen verwendete Begriff Selektivität ist wie folgt definiert:

$$S_i = \frac{\text{mol MeOH umgesetzt zu Produkt i x 100}}{\text{mol MeOH umgesetzt total}}$$

Außerdem werden die nachstehenden Abkürzungen benutzt:

$PØ_3$ Triphenylphosphan
DPPM 1,3-Bis(diphenylphosphino)methan
DPPE 1,3-Bis(diphenylphosphino)ethan
DPPP 1,3-Bis(diphenylphosphino)propan
DPPB 1,3-Bis(diphenylphosphino)butan
MeOH Methanol
EthOH Ethanol
PrOH Propanol
BuOH Butanol
$S_{ROH}$ Summe der Selektivitäten $S_{EthOH} + S_{PrOH} + S_{BuOH}$
$S_{ROH^*}$ $S_{EthOH} + S_{prOH} + S_{BuOH} + S_{Acetale}$
HSP höher siedende Produkte
"$RuI_2$" $RuI_2(CO)_2(PØ_3)_2$
"$RuCl_2$" $RuCl_2(CO)_2(PØ_3)_2$
$Ru(acac)_3$ Rutheniumacetylacetonat
THF Tetrahydrofuran

Beispiele 1 bis 7

Die Beispiele 1 bis 7 geben den Stand der Technik wieder. Sie beschreiben die Umsetzung von Methanol mit Synthesegas in Gegenwart ein- oder zweizähnige Phosphane enthaltender Katalysatoren, jedoch ohne Zusatz von Dioxan zum Reaktionsgemisch. Die Umsetzungsprodukte enthalten bei Einsatz von Triphenylphosphan einen sehr hohen, bei Einsatz zweizähniger Phosphane immer noch einen bedeutenden Anteil Ether.

Tabelle 1

| Beispiel Nr. | 1 | 2 | 3 | 4 | 5 | 6 | 7 |
|---|---|---|---|---|---|---|---|
| Temp. (°C) | 200 | 200 | 200 | 200 | 200 | 200 | 200 |
| Druck (MPa) | 40 | 50 | 50 | 50 | 40 | 50 | 50 |
| Phosphan | PØ3 | PØ3 | DPPM | DPPE | DPPP | DPPP | DPPB |
| - Menge(mmol) | 0,2 | 0,2 | 0,2 | 0,2 | 0,2 | 0,2 | 0,2 |
| Zeit (h) | 3 | 3 | 3 | 3 | 3 | 3 | 3 |
| Produkte | Selektivität $S_i$ (%) | | | | | | |
| Ether | 23 | 23 | 13 | 9 | 12 | 12 | 18 |
| Ester | 10 | 11 | 13 | 15 | 10 | 8 | 7 |
| Acetale | 0 | 0 | 9 | 11 | 7 | 3 | 6 |
| HSP | 0 | 0 | 2 | 5 | 0 | 3 | 0 |
| EthOH | 61 | 58 | 53 | 48 | 59 | 60 | 60 |
| PrOH + BuOH | 6 | 8 | 10 | 12 | 10 | 14 | 9 |
| $S_{ROH}$* | 67 | 66 | 72 | 71 | 76 | 77 | 75 |
| Umsatz (%) | 77 | 83 | 81 | 87 | 85 | 91 | 78 |

Beispiele 8 bis 12

Die Beispiele 8 bis 12 werden unter Zusatz wechselnder Mengen Dioxan durchgeführt. Die Etherbildung nimmt drastisch ab, die Selektivität der Reaktion hinsichtlich der Entstehung von Alkoholen wird deutlich erhöht.

Tabelle 2

| Beispiel Nr. | 8 | 9 | 10 | 11 | 12 |
|---|---|---|---|---|---|
| Temp. (°C) | 200 | 200 | 220 | 220 | 220 |
| Druck (MPa) | 50 | 50 | 50 | 50 | 50 |
| Dioxan (mmol) | 100 | 100 | 100 | 200 | 300 |
| DPPP (mmol) | 0,2 | 0,2 | 0,2 | 0,2 | 0,2 |
| Zeit (h) | 3 | 6 | 6 | 6 | 6 |
| Produkte | Selektivität $S_i$ (%) | | | | |
| Ether | 3 | 6 | 7 | 2 | 2 |
| Ester | 12 | 6 | 3 | 2 | 2 |
| Acetale | 23 | 3 | 1 | 0 | 2 |
| HSP | 0 | 4 | 0 | 0 | 0 |
| EthOH | 51 | 43 | 56 | 81 | 81 |
| PrOH + BuOH | 11 | 38 | 33 | 15 | 13 |
| $S_{ROH}$* | 85 | 84 | 90 | 96 | 96 |
| Umsatz (%) | 88 | 100 | 98 | 85 | 80 |

Beispiele 13 bis 18

In den Beispielen 13 bis 18 wird die Aktivität unterschiedlicher Ru-verbindungen untersucht. Dem Standardansatz werden 200 mmol Dioxan und 0,2 mmol DPPP zugesetzt; die Reaktion erfolgt bei 220 °C und 50 MPa Druck; sie wird nach 6 h unterbrochen.

Die Versuche zeigen, daß sich die Rutheniumverbindungen hinsichtlich ihrer Aktivität nicht wesentlich unterscheiden.

Tabelle 3

| Beispiel Nr. | 13 | 14 | 15 | 16 | 17 | 18 |
|---|---|---|---|---|---|---|
| Ru-verbindg. | "$RuI_2$" | "$RuCl_2$" | $RuCl_3.3H_2O$ | | Ru(acac)$_3$ | |
| Produkte | Selektivität $S_i$ (%) | | | | | |
| Ether | 2 | 3 | 4 | 4 | 2 | 3 |
| Ester | 2 | 3 | 0,5 | 2 | 1 | 2 |
| Acetale | 0 | 2 | 1,5 | 2 | 0 | 0 |
| HSP | 0 | 0 | 0 | 0 | 0 | 0 |
| EthOH | 81 | 75 | 84 | 80 | 86 | 78 |
| PrOH + BuOH | 15 | 17 | 10 | 13 | 11 | 17 |
| $S_{ROH}$ | 96 | 92 | 94 | 93 | 97 | 95 |
| Umsatz (%) | 85 | 87 | 71 | 82 | 75 | 88 |

Beispiele 19 bis 26

In den Beispielen 19 bis 26 wird der Einfluß des Verhältnisses $CoI_2$/DPPP auf den Anteil der Alkohole im Reaktionsprodukt untersucht. Die Umsetzung erfolgt bei 220 °C und 50 MPa unter Zusatz von 200 mmol Dioxan; sie wird nach 6 h unterbrochen.

Durch Erhöhung von $CoI_2$/DPPP von 1/1 auf 3/1 läßt sich das Verhältnis $S_{EthOH}/(S_{PrOH} + S_{BuOH})$ von 5,4 bis auf 0,5 erniedrigen. Gleichzeitig erniedrigt sich $S_{PrOH}/S_{BuOH}$ von 8,8 auf 3,7.

Tabelle 4

| Beispiel Nr. | 19 | 20 | 21 | 22 | 23 | 24 | 25 | 26 |
|---|---|---|---|---|---|---|---|---|
| $CoI_2$ (mmol) | 0,2 | 0,2 | 0,3 | 0,4 | 0,6 | 0,4 | 0,4 | 0,4 |
| DPPP (mmol) | 0,1 | 0,2 | 0,2 | 0,2 | 0,2 | 0,2 | 0,3 | 0,4 |
| Produkte | Selektivität $S_i$ (%) | | | | | | | |
| Ether | 6 | 2 | 7 | 13 | 17 | 13 | 10 | 6 |
| Ester | 2 | 2 | 4 | 4 | 3 | 4 | 3 | 2 |
| Acetale | 0 | 0 | 1 | 1 | 3 | 1 | 1 | 2 |
| EthOH | 76 | 81 | 64 | 37 | 26 | 37 | 43 | 55 |
| PrOH+BuOH | 16 | 15 | 24 | 45 | 51 | 45 | 43 | 35 |
| $S_{ROH}$ | 92 | 96 | 88 | 82 | 80 | 82 | 86 | 90 |
| $\frac{S_{EthOH}}{(S_{ProH} + S_{BuOH})}$ | 4,7 | 5,4 | 2,7 | 0,8 | 0,5 | 0,8 | 1,0 | 1,6 |
| $\frac{S_{PrOH}}{S_{BuOH}}$ | 7,6 | 8,8 | 8,7 | 4,9 | 3,7 | 4,9 | 5,3 | 6,9 |
| Umsatz | 92 | 85 | 97 | 100 | 100 | 100 | 99 | 99 |

In den Beispielen 28 und 30 wird als cyclischer Ether THF eingesetzt und seine Wirksamkeit mit der von Dioxan (Beispiele 27 und 29) verglichen.

Tabelle 5

| Beispiel Nr. | 27 | 28 | 29 | 30 |
|---|---|---|---|---|
| Temp. (°C) | 200 | 200 | 200 | 200 |
| Druck (MPa) | 40 | 40 | 40 | 40 |
| Phosphan | DPPP | DPPP | DPPP | DPPP |
| - Menge (mmol) | 0,2 | 0,2 | 0,2 | 0,2 |
| cycl. Ether | Dioxan | THF | Dioxan | THF |
| - Menge (mmol) | 100 | 100 | 100 | 100 |
| Zeit (h) | 3 | 3 | 6 | 6 |
| Produkt | Selektivität $S_i$ (%) | | | |
| Ether | 2,9 | 2,8 | 6,8 | 4,7 |
| Ester | 15,3 | 2,4* | 3,2 | 2,0* |
| Acetale | 30,1 | 41,2 | 5,4 | 4,1 |
| HSP | 2,0 | 0 | 4,5 | 4,8 |
| EthOH | 38,4 | 44,2 | 55,8 | 59,2 |
| PrOH + BuOH | 11,3 | 9,4 | 24,3 | 25,2 |
| $S_{ROH}$ | 49,7 | 53,6 | 80,1 | 84,4 |
| Umsatz (%) | 87,3 | 80,2 | 100 | 95,3 |

* Essigsäureethylester nicht bestimmt

Unter den angewandten Reaktionsbedingungen ergibt THF etwas bessere Selektivitäten bezüglich $S_{ROH}$ jedoch etwas niedrigere Umsätze als Dioxan.

Beispiele 31 und 32

In den Beispielen 31 und 32 wird das Verhalten des Katalysatorsystems bei wiederholter Verwendung ohne und mit Zusatz von $CoI_2$ untersucht. Zum Standardansatz werden 0,2 mmol DPPP und 200 mmol Dioxan gegeben. Die Reaktion erfolgt bei 220°C und 50 MPa Druck; sie wird nach 6 h unterbrochen. Die Ergebnisse der Einzelversuche werden in Tabelle 6 mit denen des Beispiels 11, Tabelle 2 verglichen.

Tabelle 6

| Beispiel Nr. | 11 | 31 | 32 |
|---|---|---|---|
| Versuch | 1 | 2 | 2 |
| $CoI_2$-Zugabe (mmol) | 0 | 0 | 0 |
| Produkte | Selektivitäten $S_i$ (%) | | |
| Ether | 2 | 0 | 3 |
| Ester | 2 | 2 | 3 |
| Acetale | 0 | 20 | 7 |
| EthOH | 81 | 70 | 76 |
| PrOH + BuOH | 15 | 7 | 10 |
| $S_{ROH}$ | 96 | 77 | 87 |
| Umsatz (%) | 85 | 55 | 73 |

Die Beispiele 11, 31 und 32 werden mit demselben Katalysatorsystem durchgeführt, in Beispiel 11 (Versuch 1) ist es zum erstenmal, in den Beispielen 31 und 32 (Versuch 2) zum wiederholten Male eingesetzt. In Beispiel 31 erfolgt die Abtrennung des Katalysators an der Luft, in Beispiel 32 unter Luftausschluß. Hierzu wird die Reaktionslösung unter $N_2$ dem Autoklaven entnommen. Die Reaktionsprodukte und das Dioxan werden unter Ölpumpenvakuum bei 25°C abgezogen. Zum öligen Rückstand gibt man unter $N_2$ 20 mmol Wasser, 200 mmol Dioxan und 200 mmol Methanol. Anschließend überführt man

die Lösung, ebenfalls unter $N_2$, in den Autoklaven.

Die Beispiele zeigen, daß die Aktivität des Katalysatorsystems im Verlauf des Versuches abnimmt. Die Desaktivierung nimmt zu, wenn bei Katalysatorabtrennung und- Wiedereinsatz nicht unter Luftausschluß gearbeitet wird (Beispiel 31).

Tabelle 7

| Beispiel Nr. | 11 | 33 | 34 | 35 |
|---|---|---|---|---|
| Versuch | 1 | 2 | 3 | 4 |
| $CoI_2$-Zugabe (mmol) | 0 | 0,1 | 0,1 | 0 |
| Produkte | Selektivitäten $S_i$ (%) | | | |
| Ether | 2 | 0 | 4 | 4 |
| Ester | 2 | 1 | 4 | 2 |
| Acetale | 0 | 2 | 4 | 0 |
| EthOH | 81 | 71 | 60 | 73 |
| PrOH + BuOH | 15 | 26 | 28 | 21 |
| $S_{ROH}$ | 96 | 97 | 88 | 94 |
| Umsatz (%) | 85 | 94 | 97 | 94 |

Die Abtrennung des Katalysatorsystems von Reaktionsprodukt und Dioxan und sein Wiedereinsatz in der Synthese erfolgt wie in Beispiel 32. Insgesamt wird derselbe Katalysator viermal verwendet, in den Versuchen 2 und 3 (Beispiele 33, 34) wird jeweils $CoI_2$ zugesetzt um die Desaktivierung zu beheben. Verglichen mit Versuch 1 (Beispiel 11) werden durch die $CoI_2$-Zugabe der Umsatz erhöht und $S_{ROH}$ etwa konstant gehalten (Tabelle 7).

## Ansprüche

1.) Verfahren zur Herstellung von Ethanol im Gemisch mit Propanol und Butanol durch Umsetzung von Methanol mit Kohlenmonoxid und Wasserstoff bei Temperaturen von 180 bis 250° C und Drucken von 20 bis 60 MPa in Gegenwart von 0,07 bis 0,4 Mol Wasser je Mol Methanol und von Katalysatoren, die Kobalt und Ruthenium in elementarer Form oder in Form von Verbindungen, Jod oder Jodid und ein organisches Monophosphan oder Phosphit oder ein zweizähniges organisches Phosphan oder Phosphit enthalten, dadurch gekennzeichnet, daß dem Reaktionsgemisch je Mol Methanol 0,2 bis 4 Mol eines cyclischen Mono- oder Polyethers zugesetzt werden.

2.) Verfahren nach Anspruch 1, dadurch gekennzeichnet, daß man 0,3 bis 3 Mol und insbesondere 0,5 bis 1,5 Mol eines cyclischen Mono- oder Polyethers je Mol Methanol zusetzt.

3.) Verfahren nach Anspruch 1 oder 2, dadurch gekennzeichnet, daß der cyclische Mono- oder Polyether Tetrahydrofuran oder Dioxan ist.

4.) Verfahren nach einem oder mehreren der vorhergehenden Ansprüche, dadurch gekennzeichnet, daß als Bestandteil der Katalysatoren organische zweizähnige Phosphane oder Phosphite der allgemeinen Formel

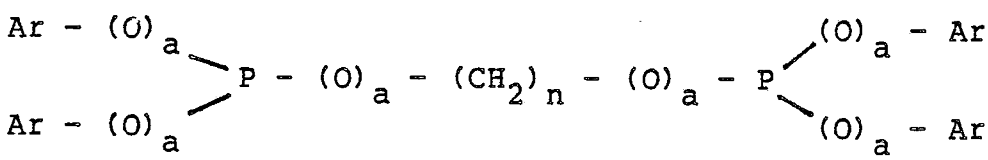

wobei Ar ein Arylrest, insbesondere der Phenylrest ist, a 0 oder 1 und n eine ganze Zahl von 1 bis 6 bedeuten.

5.) Verfahren nach Anspruch 4, dadurch gekennzeichnet, daß als organisches Monophosphan Tributylphosphan, Triphenylphosphan, Tri-p-tolylphosphan oder Trimethoxiphosphan eingesetzt werden.

6.) Verfahren nach Anspruch 4, dadurch gekennzeichnet, daß als organische zweizähnige Phosphane 1,2-Bis(diphenylphosphino)methan, 1,3-Bis(diphenylphosphino)ethan, 1,3-Bis(diphenylphosphino)propan oder 1,4-Bis(diphenylphosphino)butan eingesetzt werden.

7.) Verfahren nach einem oder mehreren der vorhergehenden Ansprüche, dadurch gekennzeichnet, daß das Reaktionsgemisch je Mol Methanol 0,07 bis 0,4 und insbesondere 0,1 bis 0,3 Mol Wasser enthält.

8.) Verfahren nach einem oder mehreren der vorhergehenden Ansprüche, dadurch gekennzeichnet, daß je g-Atom Kobalt 20 bis 10.000, vorzugsweise 50 bis 5.000 und insbesondere 200 bis 2.000 Mol Methanol eingesetzt werden.

9.) Verfahren nach einem oder mehreren der vorhergehenden Ansprüche, dadurch gekennzeichnet, daß Kobalt und Phosphan bzw. Phosphit in einem molaren Verhältnis von 1 : 0,1 bis 1 : 20, vorzugsweise 1 : 0,1 bis 1 : 5 und insbesondere 1 : 0,33 bis 1 : 2 eingesetzt werden.

10.) Verfahren nach einem oder mehreren der vorhergehenden Ansprüche, dadurch gekennzeichnet, daß das Atomverhältnis von Kobalt zu Ruthenium 1 : 0,0005 bis 1 : 1, vorzugsweise 1 : 0,05 bis 1 : 0,5 und insbesondere 1 : 0,1 bis 1 : 0,3 beträgt.

11.) Verfahren nach einem oder mehreren der vorhergehenden Ansprüche, dadurch gekennzeichnet, daß in den Katalysatoren je g-Atom Kobalt 0,002 bis 3, vorzugsweise 0,1 bis 2,5 und insbesondere 0,5 bis 2 Mol Jod enthalten sind.

12.) Verfahren nach einem oder mehreren der vorhergehenden Ansprüche, dadurch gekennzeichnet, daß das molare Verhaltnis von Methanol zu Synthesegas 1 : 0,1 bis 1 : 20, vorzugsweise 1 : 0,5 bis 1 : 5 beträgt.

13.) Verfahren nach einem oder mehreren der vorhergehenden Ansprüche, dadurch gekennzeichnet, daß dem Katalysator bei wiederholtem Einsatz, nach Abtrennung vom Reaktionsprodukt, jeweils 0,2 bis 5 Gew.-%, vorzugsweise 0,5 bis 1 Gew.-% des ursprünglich als Metall oder Verbindung eingesetzten Kobalts zugefügt werden.

14.) Verfahren nach einem oder mehreren der vorhergehenden Ansprüche, dadurch gekennzeichnet, daß die Umsetzung bei Temperaturen von 180 bis 250 °C, vorzugsweise 180 bis 230 °C und insbesondere 200 bis 220 °C und Drücken von 20 bis 60 MPa, vorzugsweise 45 bis 60 MPa und insbesondere 40 bis 50 MPa erfolgt.